# EUROPEAN PATENT SPECIFICATION

(11) **EP 1 083 907 B1**
(45) Date of publication and mention of the grant of the patent: **10.09.2003**
(21) Application number: 99921041.2
(22) Date of filing: 31.05.1999
(51) Int. Cl.: A61K 31/70, A61K 31/17, A01N 47/34, A01N 43/90

(54) **CONTROL OF PARASITIC INFESTATIONS IN FARMED AND WILD FISH**
BEKÄMPFUNG VON PARASITISCHEN BEFALLEN IN FISCHEN
LUTTE CONTRE LES INFECTIONS PARASITAIRES DES POISSONS D'ELEVAGE OU SAUVAGES

(30) Priority: 09.06.1998 NO 982650
(43) Date of publication of application: 21.03.2001
(60) Divisional application: 03075182.0
(73) Proprietor: Alpharma AS, 0212 Oslo (NO)
(72) Inventor: ALEXANDERSEN, Svein, N-4024 Stavanger (NO); EVENSEN, ystein, N-0198 Oslo (NO); SYVERTSEN, Christian, N-1900 Fetsund (NO); MARTINSEN, Bernt, N-1386 Asker (NO)
(74) Representative: Plougmann & Vingtoft A/S
(86) International application number: IB9900966
(87) International publication number: WO99063824

(56) References cited:
- EP-A- 0 750 907
- WO-A-92/08352
- WO-A-96/11707
- WO-A-96/41536
- WO-A-97/46204
- UMEHARA O ET AL: "COMPATIBILIDADE ENTRE DORAMECTIN E VACINA CONTRA A FEBRE AFTOSA ADMINISTRADOS SIMULTANEAMENTE EM BOVINOS" REVISTA BRASILEIRA DE PARASITOLOGIA VETERINARIA - BRAZILIAN JOURNAL OF VETERINARY PARASITOLOGY, SAO PAULO, BR, vol. 2, no. 2, 1993, pages 141-144, XP000653981 ISSN: 0103-846X

## Description

### FIELD OF INVENTION

The present invention pertains in its broadest aspect to the field of controlling diseases in fish and in particular there are provided means for controlling parasitic infestations in fish such as farmed fish and more specifically, novel compositions for treating and/or preventing infestations with sea lice and other crustacean fish parasites are provided, including injectable compositions for such treatment and/or prevention.

### TECHNICAL BACKGROUND AND PRIOR ART

Parasitic infestations constitute considerable problems in the fish farming industry as well as in wild fish. This applies especially to farmed fish in fresh water and seawater. Damages due to parasitic infestations result in considerable losses and increased workloads for the fish farmers. Infestation with sea lice (*Lepeophtheirus salmonis* and *Caligus elongatus*) is considered to be one of the most important disease problems in the farming of salmonids, especially in Atlantic salmon (*Salmo Salar*) and rainbow trout (*Oncorhynchus mykiss*). In addition to the costs that are associated with treatment, lower classification ratings of slaughtered fish and reduced growth rate due to reduced feed intake contribute to the economic losses for the fish farmer.

In addition to the damages caused in farmed fish, recent research has shown that sea lice could be the most important single cause leading to weakening of several wild salmon stocks. The emigration of salmon smolt from river systems is often coincidental with rising seawater temperatures in the fjord and coastal areas, which leads to a massive attack of copepodites (sea lice larvae) on the smolt. An attack of 40 or more sea lice on salmon smolt is fatal to fish weighing less than 25 grams, and in several regions in Norway premature return of post-smolt sea trout has been observed. The decline in the stock of salmonids that have been documented in several Norwegian salmon rivers over the past few years, could be related to the fact that the amount of sea lice in connection with fish farming has increased.

Up till now, the most common treatment of fish parasites involves bathing or immersing the fish in a treatment solution comprising an antiparasitically active compound. This includes both skin and gill parasites. Bathing in formalin is a widespread treatment against many parasites, especially in fresh water, while bathing in organophosphates (metrifonate, dichlorvos, azamethiphos), pyrethroids (pyrethrum, cypermethrin, deltamethrin) or hydrogen peroxide are the most common bath treatments against e.g. sea lice (*Lepeophtheirus salmonis, Caligus elongatus*). These substances act directly on the parasites via the water, and a possible absorption of the active substance into the fish itself is unimportant for the effect of the active substances on the parasite.

Substances that are effective against parasitic infestations for oral administration have also been tested in fish. Substances such as chitin synthesis inhibitors, diflubenzuron and teflubenzuron, and ivermectin are examples of substances, which, if administered orally, can be effective against parasitic diseases in fish. In addition to the substances mentioned above, wrasse (*Labridae*) has been used extensively to keep sea lice infestations under control. Hexaflumuron is an insect growth regulator also a chitin synthesis inhibitor that has been shown to be non-leathal for fish (Su, Nan-Yao et al J. Economic Entomology Vol.86. No.5, 1993 & Vasuki, V. Indian J. of Exp. Biol. Vol.30, No.12, 1992).

Substances for bath treatment of parasitically infestated fish, such as sea lice infestations, must be mixed into the water where the fish normally swims. Some of the substances that are used are water-soluble (azamethiphos), while others are not (cypermethrin, deltamethrin) and therefore, the latter group of substances remain as a suspension or emulsion in water. For fish that are kept in tanks, the bath treatment can be carried out by adding the substance formulations directly into the tank where the fish are kept. For treatment of fish that are kept in sea cages, the bottom of the cage is raised manually to reduce the treatment volume. A tarpaulin is placed around the cage so that the fish are completely enclosed, whereupon the treatment substance is added to the cage. The fish swim in the solution for 20-60 minutes, depending on the treatment substance. Oxygen must be supplied to the cage in which the fish are treated. Treatment with a bottomless tarpaulin, the so-called skirt treatment, is also used to a large extent. Since the treatment volume in such cases can not be defined exactly, a larger amount of active substance will have to be used in this route of administration than is the case when a closed tarpaulin is used. It is also becoming more common to carry out bath treatments in well boats.

Organophosphates and hydrogen peroxide are only effective against the pre-adult and adult stages of sea lice (the last 3 stages of the total of 8 stages which exist on the skin of salmonids), while pyrethroids and ivermectin also have a more or less well-defined effect against the other 5 stages. None of these substances protect against new infestations after the treatment has been completed.

Hydrogen peroxide is corrosive, and must therefore be handled with great care. Transport of hydrogen peroxide requires certain precautionary measures, as it is defined as hazardous goods in great quantities. The organophosphates are toxic to humans and must therefore be treated with caution. The organophosphates could be absorbed through the skin and lead to poisoning. The therapeutic margin for organophosphates and hydrogen peroxide is small. Fish mortality has been reported on several occasions, due to overdosing of such drugs.

Pyrethroids have, in addition to their effect against pre-adult and adult lice, also an effect against the attached stages. They are not acutely toxic to the user, but is a drug group that is toxic to fish, especially small fish. Possible overdosing and increased mortality is thus possible.

All drug groups mentioned above are administered via bath treatments. This is labour-intensive and might also be a stress factor to the fish.

In addition to bath treatments, oral treatments for parasite control in fish have been developed. Two chitin synthesis inhibitors, diflubenzuron and teflubenzuron, have been documented for use against sea lice in salmon. The macrocyclical lactone, ivermectin, is also being used against parasites in salmonids in Chile, Ireland and Scotland.

Diflubenzuron and teflubenzuron, which belong to the same substance group as hexaflumuron, act by inhibiting the production of chitin which is an important part of the cuticle of insects and crustaceans. At each moulting or ectdysis, a new synthesis of chitin is required for development. If this synthesis is inhibited, the development of the insect or crustacean will be halted, and the animal under development will die. In principle, chitin synthesis inhibitors will be effective against all organisms containing chitin. Fish and mammals do not contain chitin and will therefore not be affected by substances within this drug group. This is reflected in very low toxicity for fish and mammals, including humans. Diflubenzuron and teflubenzuron are administered to the fish via the feed, absorbed and distributed to skin and mucus, where the concentration will be high enough to inhibit the development of the parasite. The disadvantage of diflubenzuron and teflubenzuron is that these substances have no effect against adult stage of parasites which do not actively synthesise chitin. Neither do they have any effect beyond the period of treatment, as attack by new parasites may occur within days of completing the treatment. This is due to the fact that the substances are eliminated relatively fast so that the concentration of the substance ends up below therapeutical level in skin and mucus. The treatment must therefore be repeated if there is a continuous risk of parasite infection from the surroundings which, under normal circumstances, is often the case. Teflubenzuron has been described as an agent and a substance for use to combat fish parasites in farmed fish and particularly salmon by administrating it through the feed (WO 96/41536).

Ivermectin impairs the transfer of neural impulses in insects and crustaceans. This leads to paralysis and death. Mammals and fish are also affected by ivermectin. However, the same transfer mechanisms that are affected in insects, are only found in the brain of fish and mammals. Mammals have an advanced blood-brain barrier which prevents toxic effect from lower concentrations. The blood-brain barrier in fish is less developed and this causes a substantial transition to the brain of ivermectin in treated fish, and toxic symptoms are found at relatively low concentrations. Ivermectin, however, is effective for treatment of parasites in fish, provided that precautions with the dosing are taken. Toxic effects and mortality may rise if the fish are overdosed. Ivermectin is often dosed 1 or 2 times a week to control sea lice infestation in salmonids. This means that the substance must be added on a continuous basis to maintain therapeutic effect and keep the fish reasonably free of lice. Ivermectin is eliminated at a slow rate which means that the effect of the treatment is maintained for 2 to 3 weeks after the treatment has been completed. This also means, however, that the treatment involves a long withdrawal period before the fish may be slaughtered and consumed. Ivermectin has not been documented and approved for use on fish in any country. Sufficient documentation about withdrawal periods, toxicity to fish and to the marine environment is therefore not available.

As it appears from the above summary of the state-of-the-art, there is an industrial need for improved means of controlling parasitic infestations in fish, which are antiparasitically effective and non-toxic to the fish and can be administered in an industrially convenient manner and which protect the fish for an extended period of time after administration. Such improvement that are provided by the invention is the treatment of fish to cure or prevent parasitic infestations by administering hexaflumuron and the administration of antiparasitically active substances by injection. Such an administration route has not been used previously, as it has hitherto been considered labour intensive as well as stressful for the fish and therefore, unsuitable.

### SUMMARY OF THE INVENTION

Accordingly, the invention pertains in a first aspect to hexaflumuron for controlling parasitic infestations in fish.

In further aspects the invention relates to the use of hexaflumeron in the manufactur of a composition for controlling parasitic infestations such as infestations with crustacean parasites including sea lice and isopod species, in fish, the use comprising administering an antiparasitically effective amount of hexaflumuron to the fish, a composition for the treatment and/or prophylaxis of parasitic infestations in fish, the composition comprising hexaflumuron and to the use of hexaflumuron in the manufacture of a composition for treatment and/or prophylaxis of parasitic infestations in fish. In accordance with the invention, hexaflumuron can be administered to the fish orally, by adding it to the habitat of the fish or by injection.

In one useful embodiment, the injectable composition contains, as a further active substance, an antigen conferring upon administration to the fish active immunological protection against viral and/or bacterial infections.

In the context of the invention, fish in which parasitic infestations are to be controlled include salmonid species such as Atlantic salmon (*Salmo salar*), rainbow trout (*Oncorhynchus mykiss*) and sea trout (*Salmo trutta*), and sea bass (*Dicentrarchus labrax).*

### DETAILED DISCLOSURE OF INVENTION

A major objective of the present invention has been to find substances and compositions for the treatment and/or prevention of infestations in fish with parasites, especially sea lice, which do not have the disadvantages of other known substances and which also protect treated fish against infections for some time after the treatment has been completed.

The invention also concerns the use of chemical substances for the manufacture of compositions comprising hexaflumuron for injection into fish which are useful to treat and protect against parasites, especially sea lice. Particularly interesting is the use of antiparasitically active substances in mixtures with vaccine components, for the manufacture of a composition that gives active immunological protection against bacterial and viral diseases as well as conferring prophylactic protection against parasites, especially sea lice. Combining vaccine and prophylactic treatment in one product results in protection against bacterial, viral and/or parasitic diseases. A combined product like this will neither cause additional stress to the fish nor increased workload for the fish farmer, as the use of injection vaccines against bacterial and viral diseases is already well established in the fish farming industry.

Injectable compositions comprising hexaflumuron according to the invention can be formulated as a solution, suspension or emulsion of the antiparasitically active substance, with or without vaccine components.

The use of injectable compositions comprising hexaflumuron to treat and protect fish against parasitic infestations will, to a large extent, eliminate the environmental problems of today, which are associated with the use of chemical substances to control parasite problems in fish. The use of injectable compositions may cause spills to the environment in the form of secretions of the active substance from injected fish. However, this kind of spill is very limited compared to the spills from feed, faeces from oral treatment and spills directly from bath solutions after bath treatment.

In addition, the invention relates to the use of the chitin synthesis-inhibiting compound, hexaflumuron, in the manufacturing of a formulation for control of fish parasites, especially lice, in salmonids. The formula for hexaflumuron is:

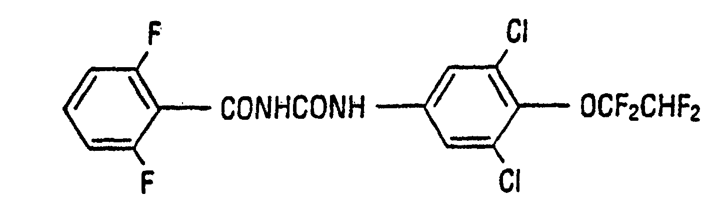

Hexaflumuron can be added to pre-manufactured fish feed or pellets or it can be mixed with the other components in fish feed before making pellets. Hexaflumuron may also be added as capsules or with other vehicles that the fish eats, for example Akvaletter™ (fish feed tablets).

The active substance, hexaflumuron, may also be prepared as a formulation that can be added directly to the water where the fish swim. The formulation may then be a solution of the active substance, a suspension of the active substance, an emulsion containing the active substance, or the active substance as a solid formulation (e.g. in the form of a powder or a granulate). The active substance can also be formulated for injections as a solution, suspension or emulsion composition of the active substance.

Irrespective of how the hexaflumuron composition is administered, the fish will absorb the active substance, and therapeutic concentration of the substance will be maintained for a certain time. This concentration is high enough to prevent further development of parasites and to prevent new parasites to attach and develop for some time after treatment. The protection period depends on formulation, route of administration and depletion rate from the fish. The depletion rate will depend on the fish species, water temperature and formulation of the substance (especially for injection). Trials with hexaflumuron show that the protection period for both oral and bath administration will be at least 8 weeks, while an emulsion vaccine, as carrier of the active substance will give considerably longer protection.

The active form of hexaflumuron is distributed to all tissues and organs of the target fish, including mucus, skin, gills, and intestines. Parasites which are affected by hexaflumuron and which are located in tissue with an antiparasitically effective concentration, will be inhibited in their further development and die. Possible localisations of parasites are skin, gills, intestines or other internal organs.

Hexaflumuron inhibits the synthesis of chitin in insects and crustaceans and might also affect other parasites that are dependent on their own synthesis of chitin. Parasites that are dependent on chitin synthesis to develop will die within a certain time after the intake of hexaflumuron. Crustaceans will not be able to go through moulting and will subsequently die. This will for example be the case with sea lice which are dependent on chitin synthesis between each moult. Adult stages that do not have an active chitin synthesis will not be affected by hexaflumuron. In *Gyrodactylus* spp. for example, the chitin synthesis of the frontal filaments could be restrained. Hexaflumuron may also affect the quality of the eggs of the female sea lice, so that they hatch abnormally or develop abnormally with larval malformations and lack of ability to complete a normal development cycle.

It is very surprising that hexaflumuron, in addition to having a therapeutic effect, also protects fish from new attacks by juvenile parasites for an extended period of time after completing the treatment. Salmon species are protected from new establishments of sea lice for a period of at least 8 weeks such as at least 12 weeks including at least 6 months and even up to 10 months after transfer to sea water. This is in great contrast to the closely related substances, diflubenzuron and teflubenzuron, which do not protect against new establishments by sea lice (see Examples 6 and 8). As opposed to other treatments against parasites in fish, hexaflumuron can be administered as an injection (see Examples 7, 8 and 10), and protect the fish against new establishments of sea lice. This is also a substantial improvement in relation to current treatment alternatives and represents a possibility to protect valuable fish individually against parasitic infestations.

The at least 8 week post-treatment protection period is especially favourable since the need for repeated treatments will be substantially reduced. This reduces pollution by therapeutic substances in the environment and is also cost effective and labour saving for the fish farmer. If used on the wild fish populations, the invention will also give protection to emigrating wild salmon and trout smolt from rivers and river systems to their habitat in the sea water (see Example 3).

No adverse or side effects have been observed from the use of hexaflumuron in any of the trials carried out, neither from injection nor from the use of hexaflumuron as a bath solution or as oral treatment. This is in regard to all the dosages that have been examined and the various administration methods which have been used.

As it is mentioned above, the invention provides compositions which are useful for controlling infestations in fish with parasites such as parasitic crustacean species e.g. useful in the control of infestations with sea lice species including *Lepeophtheirus salmonis* and *Caligus elongatus* and isopod species including as an example *Anilocra physodes.*

The invention will now be explained in further details in the following examples that demonstrate the effect of various formulations for injection in order to obtain prophylactic protection against parasites in fish, as well as the effect of hexaflumuron, other chitin synthesis inhibitors and other antiparasitic compounds as effective therapeutic and prophylactic substances against parasites in farmed and wild fish, and in the following drawings, where:

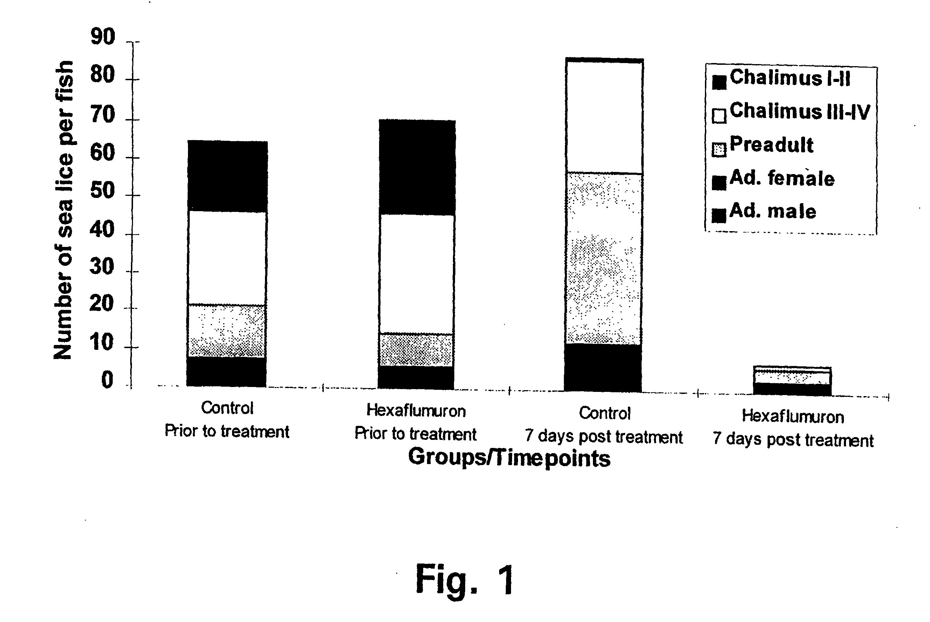

Figure 1 summarises the trial of Example 1, which was carried out on Atlantic salmon (*Salmo salar*). It shows the number of sea lice before and after treatment with hexaflumuron compared to an untreated control group. The treatment dosage was 9 mg hexaflumuron per kg fish per day for 10 days. This is indicated by "Groups/Timepoints" in the figure;

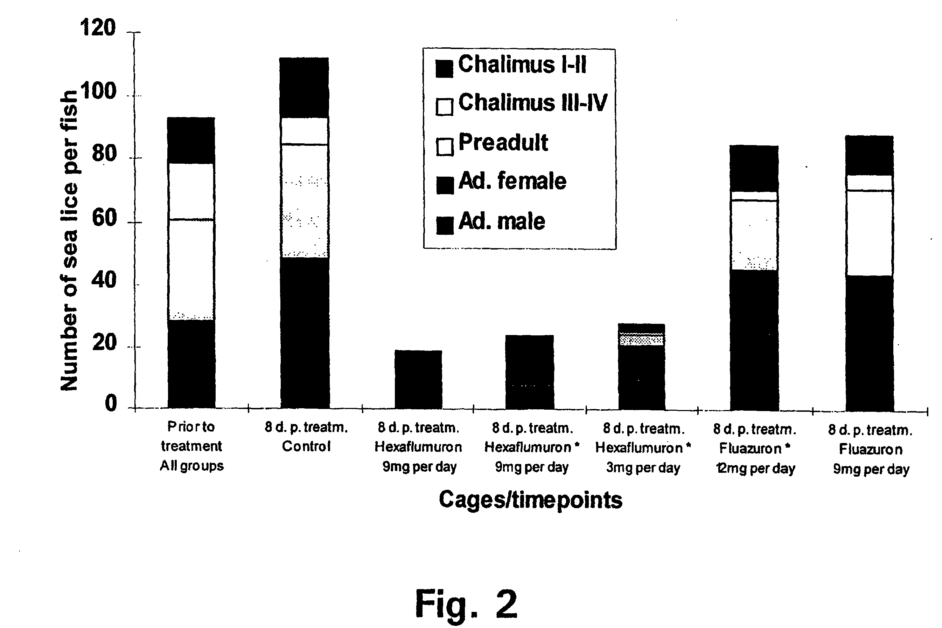

Figure 2 relates to the trial in Example 2 that was carried out on Atlantic salmon (*Salmo salar*). It shows the number of sea lice before and after treatment with hexaflumuron and fluazuron as compared to an untreated control group. The treatment dosage varied between 3 - 9 mg hexaflumuron and 9 - 12 mg fluazuron per kg fish per day for 7 days. This is indicated by "Groups/Timepoints" in the diagram. The abbreviation "8 d.p.treatm." means 8 days post treatment, and * indicates that the active substance has been administered as "Akvaletter" (fish feed tablets);

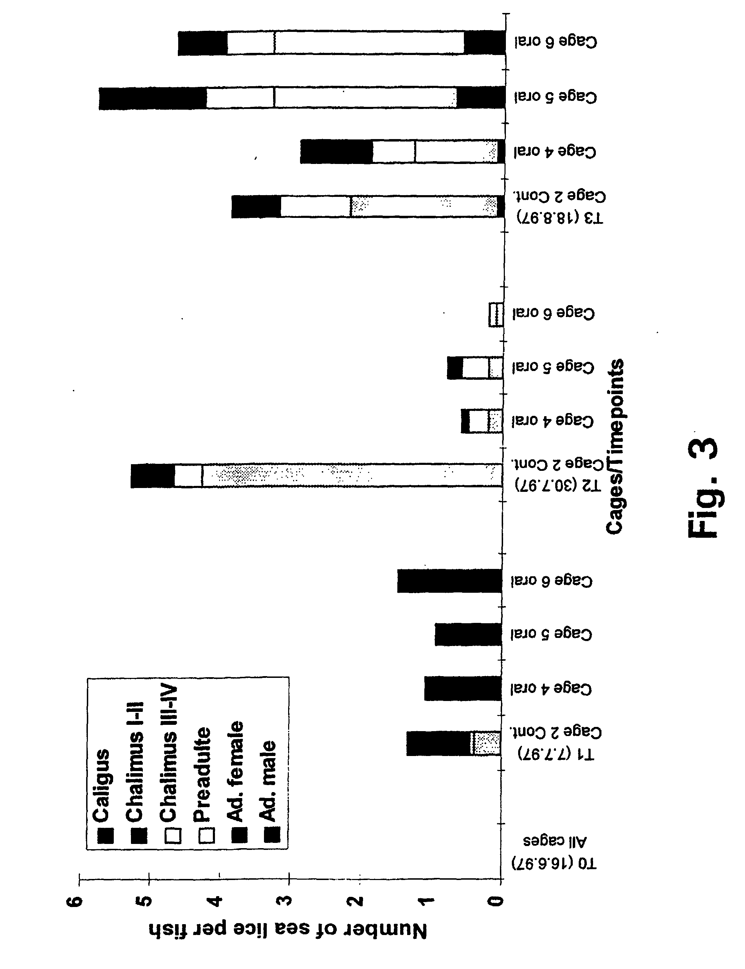

Figure 3 relates to the trial in Example 4, which was carried out on Atlantic salmon (*Salmo salar*). It shows the number of sea lice at different timepoints after oral treatment with hexaflumuron in freshwater, before sea transfer. The treated groups are compared to an untreated control group. The treatment dosage was 4 mg hexaflumuron per kg fish per day for 5 days. This is indicated by "Groups/Timepoints" in the Figure. Cage 2 = controls, Cage 4-6 = treated groups, 16.6.97 = time point for sea transfer and 5.8.97= controls treated by bathing with deltamethrin;

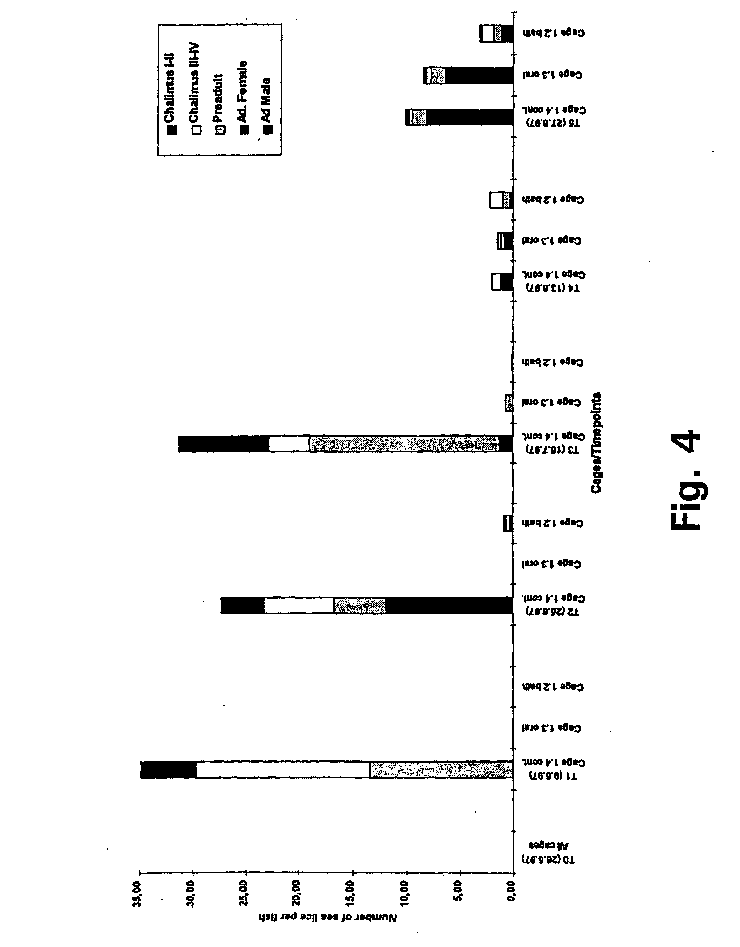

Figure 4 relates to the trial described in Example 5, which was carried out on sea trout (*Salmo trutta*). It shows the number of sea lice at different timepoints after oral or bath treatment with hexaflumuron in freshwater, before sea transfer. The treated groups are compared with an untreated control group. The dosage for oral treatment was 4 mg hexaflumuron per kilo fish per day for 5 days, and 5 ppm hexaflumuron as a 30 minute bath treatment. This is indicated by "Groups/Timepoints" in the figure. Cage 1.4 = Controls, cage 1.3 = Oral treatment, cage 1.2 = Bath treatment. The timepoints for the different periods are: 26.5.97 = sea transfer, 9.6.97 = controls treated by bath with deltamethrin after sea lice counting, 26.6.97 = controls treated by bath with deltamethrin after sea lice counting, 16.7.97 = controls treated by bath with deltamethrin after sea lice counting;

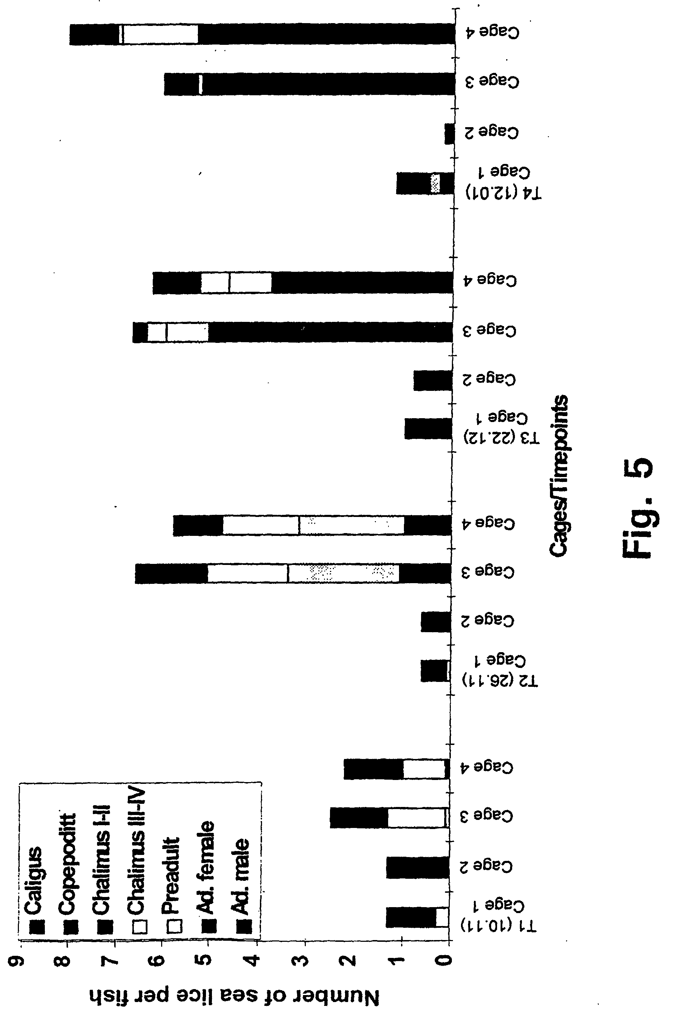

Figure 5 relates to the trial in Example 6 and shows the number of sea lice on Atlantic salmon (*Salmo salar*) at time points 1-4, after oral or bath treatment with hexaflumuron in freshwater before sea transfer. The treated groups are compared with an untreated control group, and one group that has been treated orally with teflubenzuron (10 mg/kg of teflubenzuron per kg fish per day for 7 days). The dosage for oral treatment was 5.6 mg hexaflumuron per kg fish per day for 7 days, and 5 ppm hexaflumuron as a 30 minute bath treatment. The figure shows Cage 1= oral treatment, hexaflumuron, Cage 2= bath treatment, hexaflumuron, Cage 3= oral treatment, teflubenzuron, Cage 4= controls, not treated. End of treatment: 22.10.97, Sea transfer: 23.10.97. T1 = 3 weeks post end of treatment, T2 = 5 weeks post end of treatment, T3 = 9 weeks post end of treatment, T4 = 12 weeks post end of treatment;

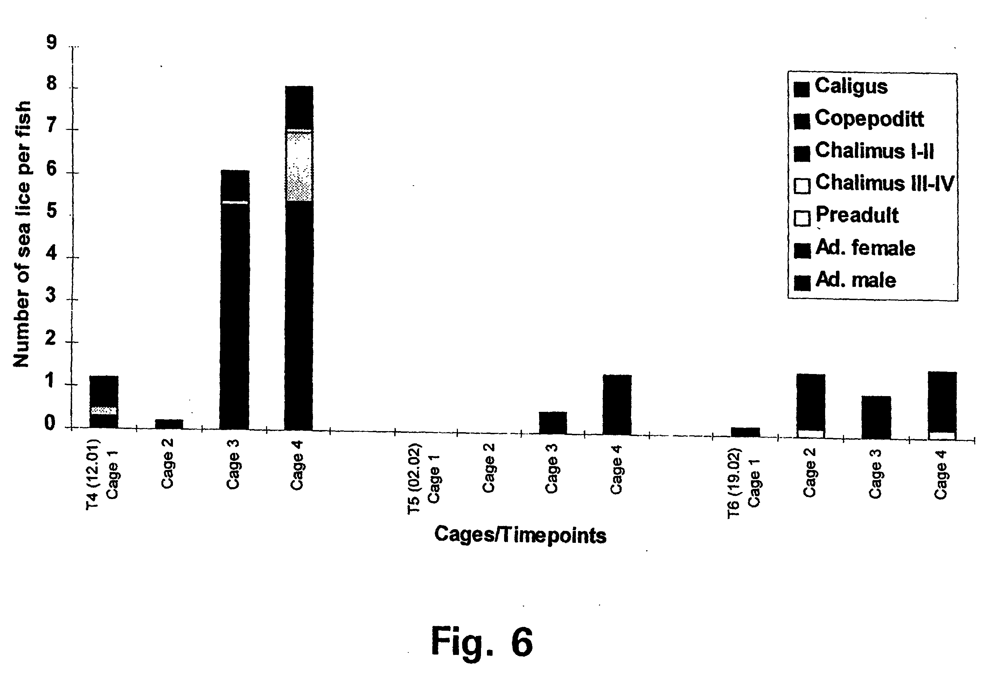

Figure 6 relates to the trial in Example 6 at time points 4-6, with the same trial parameters as for Figure 5. T5 = 15 weeks post end of treatment, T6 = 17 weeks post end of treatment. Cage 3 and 4 were treated by bath with deltamethrin after sea lice counting at T4 (12.01.98);

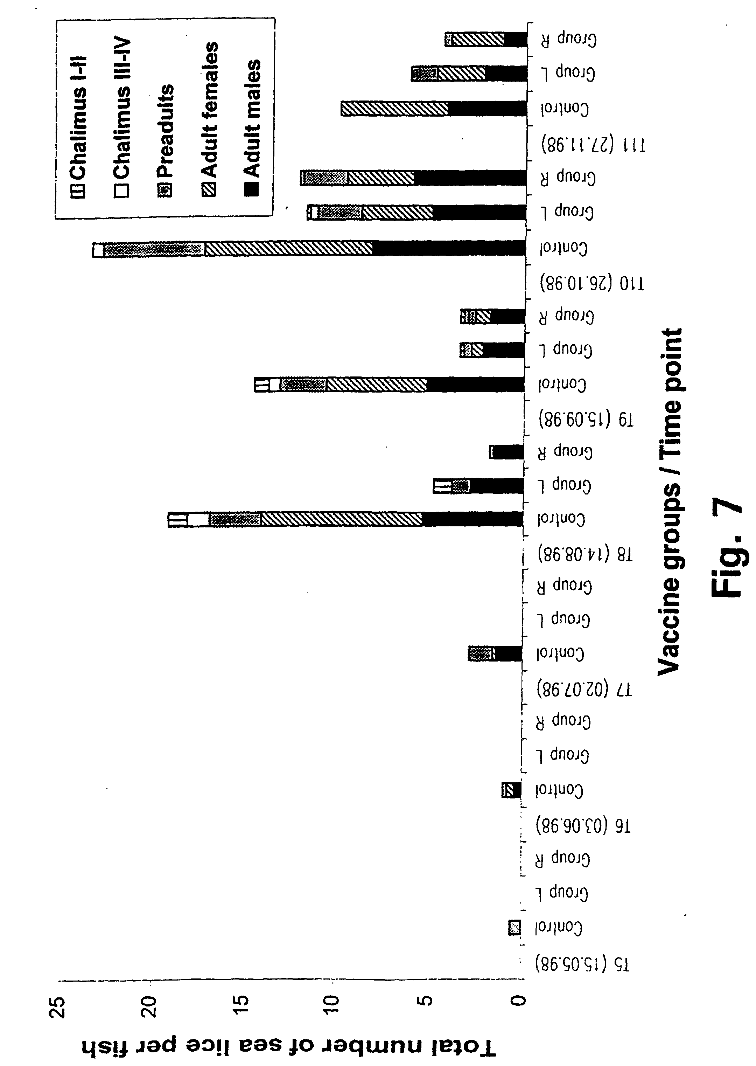

Figure 7 relates to the trial described in Example 7 where salmon were injected on 17.12.97 with two formulations (groups L and R, respectively) of hexaflumuron and a vaccine formulation without hexaflumuron (control). The figure shows the average number of sea lice at the following stages: Chalimus I-II, Chalimus III-IV, Pre-adult and Adult, throughout the trial. The vaccinated fish was transferred to sea water on 09.01.98;

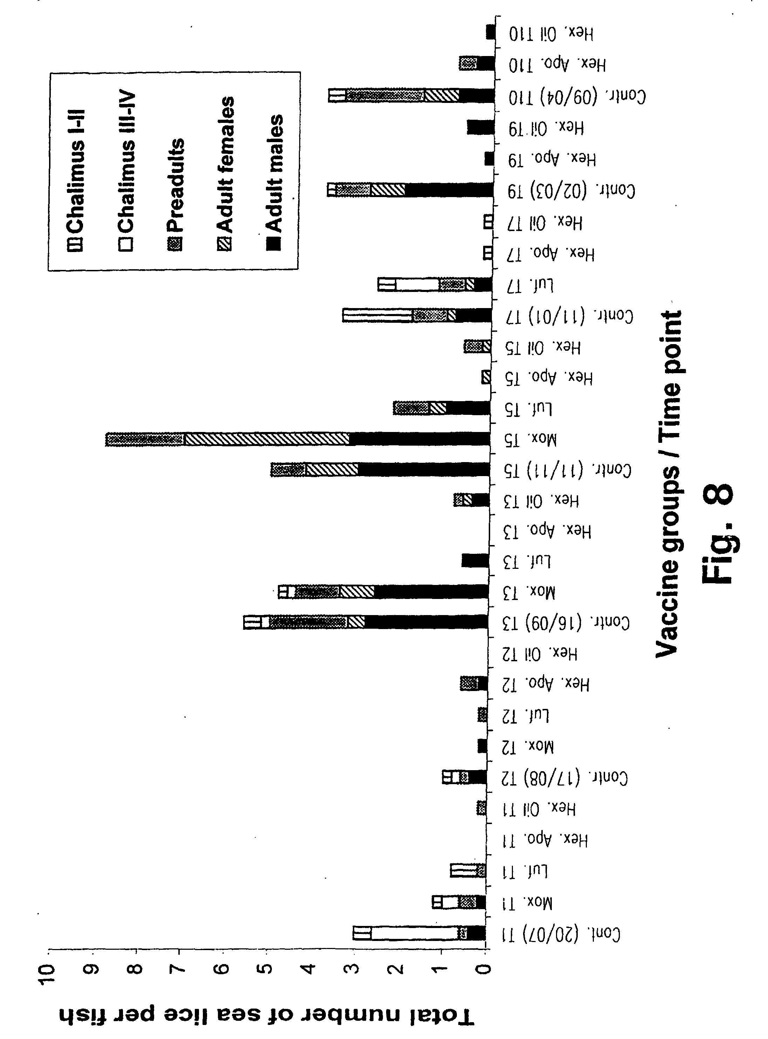

Figure 8 summarises the trial of Example 8 where salmon smolts were injected with 9 different active substances on 08.07.98. Only results for Moxidectin (Mox.), Lufenuron (Luf.) and hexaflumuron in Apoject 1-Fural (Hex.Apo) and in animal oil (Hex.oil) are shown, and

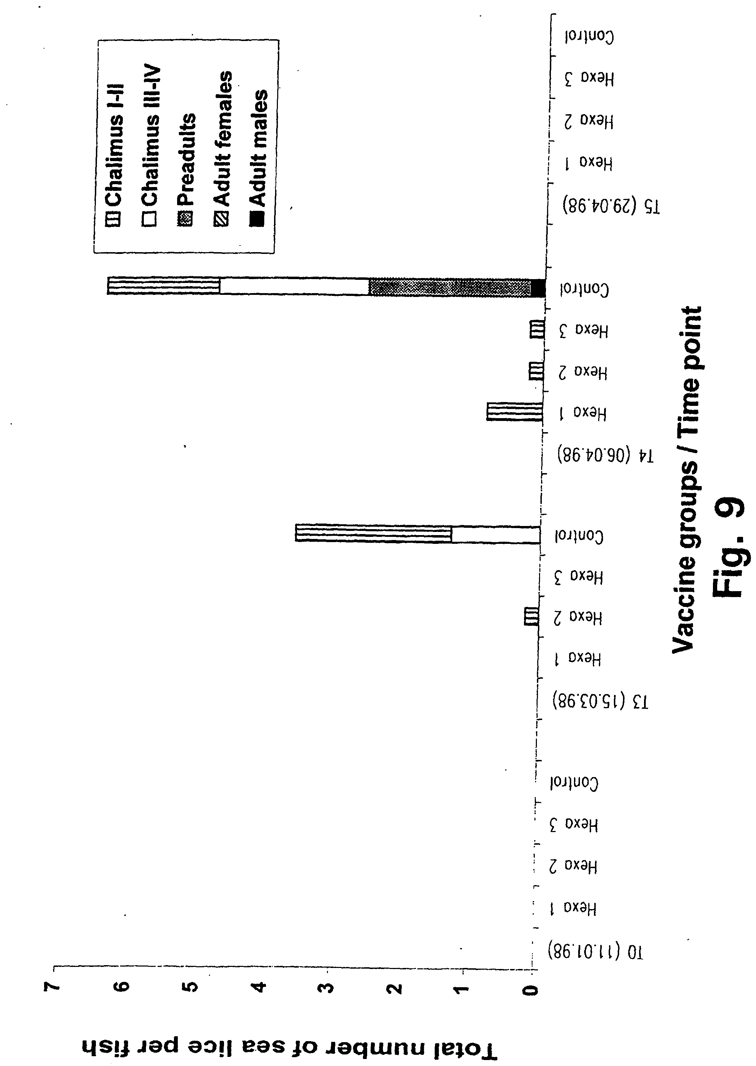

Figure 9 summarises the trial described in Example 10 where salmon were injected on 03.12.97 with 3 different formulations of hexaflumuron (Hexa 1, Hexa 2 and Hexa 3). The fish were transferred to sea water at T0.

### EXAMPLE 1

About 800 Atlantic salmon (*Salmo salar*) which weighed on average 800 grams were kept in 2 different cages, with 400 fish in each. The fish were exposed to natural infection. They were heavily infected and treatment with hexaflumuron was initiated in one of the cages, while the fish in the other cage remained untreated and were used as negative controls. In the treatment cage hexaflumuron was administered orally. The substance was coated onto the outer surface of ordinary fish pellets. Thus, the fish received a dosage of 9 mg of hexaflumuron per kg each day for 10 days via the medicated fish pellets which contained 1.5 g of hexaflumuron per kg feed. The fish were fed with these medicated pellets, following normal feeding procedures, whereas the control fish were given ordinary fish feed from T. Skretting A/S (Nutreco). The water temperature was 7.6 - 9.4°C during the trial. Figure 1 shows that the hexaflumuron treatment reduced the number of lice by more than 90% as compared to the control group. The reduction of sensitive chalimus and pre-adult stages was about 95%.

### EXAMPLE 2

About 600 Atlantic salmon (*Salmo salar*) of an average weight of 1,000 grams were kept in a minicage. The fish were exposed to natural infection. Before treatment was initiated, the fish had been exposed to an extensive sea lice infection at an average of about 93 lice per fish. Before treatment, the fish were randomly distributed to 6 different minicages with 100 fish in each. The fish in three of these cages were treated with hexaflumuron, two with fluazuron (another chitin synthesis inhibitor), and one served as negative control. The dosage in the different cages is shown in the table below.

**Table 1.**

| Dosage in treatment and control cages | | | | | |
|---|---|---|---|---|---|
| Cage number, group number | No. of fish | Formulation | Substance | Dose per day | Number of treatment days |
| 1 | 100 | Coated feed | Hexaflumuron | 9.0 mg | 7 days |
| 2 | 100 | Akvaletter | Hexaflumuron | 3.0 mg | 7 days |
| 3 | 100 | - | Control | - | - |
| 4 | 100 | Akvaletter | Hexaflumuron | 9.0 mg | 7 days |
| 5 | 100 | Akvaletter | Fluazuron | 12.0 mg | 7 days |
| 6 | 100 | Coated feed | Fluazuron | 9.0 mg | 7 days |

All the cages were treated at the same time. The fish in the control cage were given ordinary fish feed from Ewos. The water temperature was 8.0 - 9.8°C during the trial. Figure 2 shows that the hexaflumuron treatment reduced the number of lice with more than 70% as compared to the control treatment in all the groups treated with hexaflumuron. The reduction of sensitive chalimus and pre-adult stages was > 90%. For the groups given the highest dosage, the reduction was> 99%. Figure 2 also shows that the groups treated with fluazuron had a reduction of only 25% as compared to the control group.

### EXAMPLE 3

About 2,000 wild sea trout (*Salmo trutta*) were divided into two groups and marked with Carlin tags in two different colours (yellow or red) so that individuals of the groups could easily be distinguished from each other when observed in the water. The group with yellow Carlin tags were given feed containing hexaflumuron. The dose was about 3 mg of hexaflumuron per fish per day for six days. The feeding was done the last week before they were transferred to the sea in the beginning of May. The control group had red Carlin tags and was fed ordinary fish feed until sea transfer. Both groups of fish were transferred to the sea 200 metres from the river mouth of the Bondhus River in Hordaland in Norway. From sea transfer (May 8) until August 7, neighbouring rivers and estuaries round these rivers were examined every week, using fishing by application of high voltage and net fishing.

Tagged fish was not caught until June 20. From June 20 to August 7, 22 control fish and 13 treated fish were caught. The untreated fish were caught in the lower parts of the rivers and were heavily infected with sea lice. The 13 treated fish were exclusively caught in the estuary around the rivers (in sea water), none were caught in freshwater. None of the treated fish showed signs of sea lice infections.

The following average lengths and weights were recorded for the fish that were caught during the period:
Control fish: Average weight 50.4 ± 48.8 grams. Average length 19.7 ± 13.3 cm.
Treated fish: Average weight 87.1 ± 69.6 grams. Average length 35.9 ± 19.1 cm.

The results from this trial demonstrate that hexaflumuron protects sea trout smolt from sea lice infections for a period after sea transfer, a period in which the smolt is very vulnerable to sea lice infestations. Absence of sea lice in the hexaflumuron-treated group is the only logical explanation of the increased growth in this group as compared to the untreated control group.

### EXAMPLE 4

About 2,500 salmon (*Salmo salar*) of average weight 42 grams were treated orally with a daily dosage of 4 mg hexaflumuron per kilo fish for 5 days. The treatment was carried out in freshwater one week before the fish were transferred to sea water. In the sea water, the fish was distributed into 3 different minicages with 800-850 fish in each. At the same time 1,000 untreated fish (from a different fish farm) weighing on average 50 grams were transferred to sea in a neighbouring cage. After being transferred to sea water, the fish were exposed to natural sea lice infection. The number of lice on 10 fish from each cage were counted every third week to check if there was any difference in the number of lice in the cages. When ending the trial, about 12 weeks after the fish had been transferred to sea, the fish in each cage were weighed to check possible differences in growth (SGR, specific growth rate). The water temperature varied between 10-16°C during the trial. Figure 3 shows the average number of lice in each cage throughout the study. The figure also demonstrates that, 3 and 6 weeks after the fish had been transferred to sea (timepoint 1 and 2), treated fish had a significantly lower number of lice larger than Chalimus II,. From 9 weeks on, there was no difference between the groups. The dosage rate that was used in this trial, i.e. 4 mg of hexaflumuron per kg fish per day, is very low. By increasing the dosage the protection time would probably be extended. 7 weeks into the trial, the control fish were treated with a bath treatment containing deltamethrin to reduce the number of pre-adult lice. This treatment was successful and the number of lice was reduced to a level lower than or equivalent to the groups that had been treated orally before sea transfer. It was not necessary to administer a bath treatment in the cages with orally treated fish at this time. The SRG was measured after about 12 weeks and it was estimated at 1.79% in the control group, and 1.95 - 2.00% in the treated groups.

Since the control fish and treated fish came from different origins, one cannot conclude that this difference was due to fewer lice in the treated groups. It is, however, possible that a smaller number of lice did cause the better growth rate. The trial demonstrates that the hexaflumuron treatment protected the fish from sea lice infestation for 6-9 weeks after they had been transferred to sea. In this trial there was no need for bath treatment in the orally treated groups the first 9 weeks, whereas the control group had to be treated once during the same period of time.

### EXAMPLE 5

About 300 sea trout (*Salmo trutta*) of an average weight of 160 grams were divided into three groups of 100 fish each. One group was given oral treatment for one week before they were transferred to sea at a daily dosage of 4 mg hexaflumuron per kg fish for 5 days. The second group was treated with a bath treatment using a dosage of 5 ppm hexaflumuron for 30 minutes with a formulation containing 10% hexaflumuron, while the last group remained untreated. The treatments were carried out in fresh water during the last week before the fish were transferred to sea water. In the sea water, the fish were distributed into 3 mini-cages with 100 fish in each. The fish were then exposed to natural sea lice infection. Lice from 6-10 fish from each cage were counted every third week to assess whether there was any difference in the number of lice between the cages. At the end of the trial, i.e. about 13 weeks after the fish had been transferred to sea, the fish in each cage were weighed to check possible differences in SGR. The water temperature varied within the range of 12 - 22°C during the trial. Figure 4 shows the average number of lice in each cage throughout the study. The figure shows that treated fish had a significantly lower number of lice larger than Chalimus II at 2, 5 and 8 weeks, respectively after sea transfer (timepoint 1, 2 and 3).

After 11 weeks there was no difference between the groups, but the number of lice was very low in all groups, which makes it difficult to determine the length of the protection period. The dosage which was used in this trial, i.e. 4 mg hexaflumuron per kg fish per day, is very low. An increase of the dosage will probably increase the protection time. The control fish had to be treated 2, 5 and 8 weeks after they had been transferred to sea due to the high number of lice (26 - 34). A bath treatment containing deltamethrin was used for the treatment. The treatment was successful, and the number of lice was reduced to a low level. It was not necessary to administer a bath treatment to the cages which earlier had been treated with hexaflumuron. The SGR was determined after about 13 weeks. It was estimated at 0.37% in the control group and 0.84 - 0.98% in the treated groups. In addition to a high number of lice in the control group, low SGR was caused by very high temperatures and problems with fish maturation in all the groups. The difference in growth rate between the control group and the treated groups was statistically significant.

The trial shows that the hexaflumuron treatment protected the fish for at least 8 weeks after they had been transferred to sea. In this trial there was no need for bath treatment of the hexaflumuron treated groups during the 13 weeks where the trial was in progress, while the control group had to be treated three times during the same period.

### EXAMPLE 6

About 400 Atlantic salmon (*Salmo salar*) of an average weight of 80 grams were divided into four groups of 100 fish in each. One group was given an oral treatment of 5.6 mg hexaflumuron per kg fish each day for 7 days before sea transfer. Group two was given a bath treatment with a dosage of 5 ppm (mg/l) hexaflumuron for 30 minutes with a formulation containing 10% hexaflumuron. Group three was given oral treatment with a daily dosage of 10 mg teflubenzuron per kg fish for 7 days, while the last group remained untreated. All treatments were carried out in fresh water during the last week before the fish were transferred to sea water. In sea water, the fish were distributed into 4 different mini-cages with 100 fish in each cage. The fish were then exposed to natural sea lice infestation. Every third week, lice from 10 fish in each cage were counted to assess if there was a difference in the number of lice between the cages. The trial was completed about 17 weeks after the fish had been transferred to sea. The water temperature varied between 4.5 and 10.5°C during the trial.

Figures 5 and 6 show the average number of lice in each cage throughout the study. The figures show that fish treated with hexaflumuron had a significantly lower number of lice larger than Chalimus II at 5, 8 and 11 weeks (time points 2, 3 and 4). The group treated with teflubenzuron had the same number of lice as the untreated control group at each assessment, which demonstrates that teflubenzuron does not have the same protective effect as hexaflumuron. After the recording of lice at T4 (11 weeks after treatment) had been completed, the control group and the teflubenzuron treated group were treated with a bath treatment containing deltamethrin, to control the sea lice infection. The treatment was successful and the number of lice was reduced to a low level in these groups as well. The decision to treat two cages (control, teflubenzuron) was taken in co-operation with the fish farmer, and fish (untreated) in commercial cages of the farm were treated at the same time. 15 and 17 weeks (timepoints 5 and 6) after the fish had been transferred to sea, there was no difference between the groups. The number of lice was very low in all the groups at these time points (lack of new attacks), which makes it difficult to determine the exact protection period. The fish were, however, protected for at least 11 weeks.

Two days after oral treatment had been completed and at timepoint 3 (65 days after completed medication), muscle/skin tissue from five fish in group 1 was chemically analysed to check the concentration of hexaflumuron. The average concentration of hexaflumuron two days after completed medication was 4.4 µg/g tissue. 65 days after, the average concentration level was 1.2 µg/g tissue.

### EXAMPLE 7

About 80 salmon (*Salmo salar*) at an average weight of about 80 grams were divided into two groups and injected with two different formulations of hexaflumuron at a dosage of about 50 mg/kg. Both formulations were prepared based upon a vaccine against furunculosis containing inactivated cells of *Aeromonas salmonicida* subsp. *salmonicida.* Hexaflumuron was mixed/formulated into the vaccine in two different manners to study the effect of the formulation on the depot effect in the fish and on the stability characteristics of the vaccine emulsions.

The fish were dosed (injected) while they were in freshwater and 23 days after the administration they were transferred to sea water (mini-cages). In parallel with the two groups, a third group comprising 40 fish of the same origin and size was vaccinated with a conventional vaccine without hexaflumuron. This group was kept in the same tank (freshwater) and cage (sea water) as the "hexaflumuron injected" groups and served as negative control/reference group in respect of sea lice infestations. Prior to the start of the trial, the groups were marked by fin cutting. The water temperature varied between 3.8 and16.0°C throughout the trial.

Figure 7 shows the average number of lice in each group throughout the trial. No sea lice were found on the fish in this trial until time point 5 (T5), i.e. 126 days after sea water transfer. An average of 0.6 pre-adult lice on the fish was recorded in the control group and the number of sea lice increased progressively during the trial in this group. No sea lice were recorded for the two treated groups until T8, i.e. 215 days after sea water transfer. At that point in time, sea lice were found on the fish of the hexaflumuron groups, but there was constantly significantly less sea lice in these groups as compared to the control group. The average number of lice was 4.8. and 1.8, respectively in the treated groups, whereas the control group had 19.2 sea lice. At T9 and T10, respectively there was still significant differences in the number of sea lice between the treated groups and the control group. Due to the high number of sea lice in the control group at T10, all the groups were treated with the bath treatment composition ALPHA MAX (deltamethrin). This resulted in a decrease in the number of sea lice in all groups at T11. At this point in time there were still significantly less lice in the treated groups as compared to the control group. All the fish were treated once more with ALPHA MAX (deltamethrin) after the sea lice counts at T11. Following this treatment, no new sea lice infestations were found in any of the groups, most likely due to the low sea water temperatures and the ensuing low infestation pressure.

Tissue samples (muscle with skin) for chemical analysis were collected from three fish at each sample point in time throughout the entire trial period in order to monitor the concentrations of hexaflumuron. At sea water transfer, the muscle/skin concentrations were between 2 and 3 µg hexaflumuron per g of tissue. Further analyses showed a slow depletion of hexaflumuron. At T8, i.e. 238 days after vaccination, the concentration of hexaflumuron varied between 0.49 and 0.53 µg per g of tissue. At T11, i.e. 345 days after injection (322 days after sea water transfer) the muscle/skin concentration of hexaflumuron in the two groups was 0.13 and 0.11 µg/g tissue, respectively. At the same point in time, different stages of sea lice had become established on these fish. This indicates that the concentration of hexaflumuron was too low to exert a full effect against sea lice but there was still a certain effect as compared to the control group.

Based on comparisons between this experiment and experiments where hexaflumuron is administered via oral treatment or after bath treatment (see the previous trials) it is observed that the protection period is substantially extended by injection. In this trial we observed excellent protection against sea lice infestation for 7 months after sea water transfer and a partially protective effect up till 10.5 months after sea water transfer. In this trial, there was little difference in tissue concentrations of hexaflumuron between the two different vaccine formulations.

### EXAMPLE 8

In this trial 9 different active substances were tested for protective effect against sea lice. 1,200 salmon smolt were, prior the start of the trial, vaccinated with a commercial vaccine, ALPHA JECT 5100. This vaccine does not contain active substances conferring protection against sea lice.

One week after sea water transfer the salmon was divided into 12 groups and injected with different formulations of the 9 different active substances. In parallel with the 12 groups, a 13th group comprising 50 fish of the same origin and size was injected with a saline (PBS) solution without active substance. This group served as negative control/reference group in respect of sea lice infestations. The groups were marked by fin cutting and distributed randomly into 3 cages.

The active substances that were used as injection were hexaflumuron, buprofezin, diflubenzuron, fluazuron, lufenuron, ivermectin, doramectin, moxidectin and milbemycin oxime, respectively. All the fish were injected with 0.2 ml of the various injection formulations. Hexaflumuron, buprofezin, fluazuron, lufenuron and diflubenzuron were administered at a dosage of about 50 mg/kg whereas ivermectin, doramectin and moxidectin were administered at a dosage of 0.2 mg/kg (recommended dosage for mammals). Milbemycin oxime was administered at a dosage of 0.5 mg/kg (recommended dosage for mammals). All of the substances were administered as a formulation prepared on the basis of a vaccine against furunculosis (Apoject 1-Fural) comprising inactivated bacteria of *Aeromonas salmonicida* subsp. *salmonicida.* Additionally, hexaflumuron and ivermectin were administered in a formulation prepared on the basis of an animal oil (without bacterial component) and hexaflumuron was further administered in an aqueous suspension (PBS). In total, the trial included 13 different groups including 1 control group, 3 groups injected with different formulations of hexaflumuron (Apoject 1-Fural, animal oil, PBS), 2 groups injected with different formulations of ivermectin (Apoject 1-Fural, animal oil) and the remaining substances were administered in a formulation based on Apoject 1-Fural. No adverse toxic effects were observed in any of the groups injected.

Sea lice on the fish were recorded about every third to fourth week. Figure 8 shows the average number of lice in some groups throughout the trial. At T2, i.e. 40 days after injection, only the groups injected with hexaflumuron, lufenuron or moxidectin showed protection against sea lice. At T3, i.e. 70 days after injection, only fish that had been injected with hexaflumuron formulated in Apoject 1-Fural or animal oil, and fish injected with lufenuron (Apoject 1-Fural) were protected against sea lice. On average, the control group had 5.6 lice at T3, whereas it for hexaflumuron (Apoject), hexaflumuron (animal oil) and lufenuron (Apoject) was 0, 0.8 and 0.6 lice on average, respectively at the same point in time.

Fish injected with lufenuron showed good protection against lice until T5, i.e. 128 days after injection. Hexaflumuron formulated in Apoject 1-Fural or in animal oil conferred significant protection against sea lice until and including T10. i.e. 9 months after injection.

Tissue samples (muscle/skin) for chemical analysis were collected from five fish injected with the various hexaflumuron formulations at each sample point in time throughout the trial. Fish injected with hexaflumuron in an aqueous suspension (PBS) showed a very rapid depletion of active substance and at T3, the hexaflumuron concentrations were below 0.02 µg per g. These low concentrations correlate well with the parallel recordings of sea lice which showed that there was no protection against sea lice at T3. At sea water transfer, the muscle/skin concentrations in the two other hexaflumuron groups (Apoject 1-Fural, animal oil) varied between 1 and 2.5 µg hexaflumuron/g tissue. Further analyses showed a slow depletion of hexaflumuron, i.e. 0.26 and 0.27 µg/g tissue, respectively in the two groups at T8 (218 days after injection). At T8, the number of sea lice was still significantly lower in the two hexaflumuron injected groups (Apoject 1-Fural, animal oil) as compared to the control group.

In this trial, a prophylactic effect against sea lice for up till 10 months has been demonstrated in two groups of fish injected with hexaflumuron. It has also been demonstrated that injection with other active substances is capable of conferring good protection for an extended period of time following injection. In this trial this was shown for lufenuron and moxidectin.

### EXAMPLE 9

In this trial, hexaflumuron was administered orally in order to test the effect against infestations with the parasitic isopod *Anilocra physodes* in Sea bass (*Dicentrarchus labrax*).

150,000 Sea bass (*Dicentrarchus labrax*) having an average weight of 2 grams were transferred from a hatchery facility and distributed into 5 cages each containing 30,000 fish. The trial was carried out in a food fish facility in the Mediterranean where the sea water temperature varied in the range of 15.0-25.0°C throughout the trial period.

Fish in four cages were fed medicated feed at an amount corresponding to 0.5% of the total biomass per day for 6 days, corresponding to 10 mg hexaflumuron/kg fish per day. One week after the medication and subsequently every three weeks the number of parasites on the fish was monitored.

The recordings of parasites showed that there was a very low infestation pressure in the facility and very low number of parasites in the control group. One week after the termination of the medication period, no parasites were found in the treated groups whereas an average of 0.1 louse was found in the control group. Three weeks after the medication period, an average of 0.15 louse was found in the treated groups whereas the control group had an average of 0.4 louse.

Accordingly, the parasite recordings showed an effect of hexaflumuron against *Anilocra physodes* during a period of about 3 weeks after medication. The infestation pressure for the remaining part of the trial period (3 months) was very low which resulted in that significant differences in respect of parasites between the treated groups and the control group at the remaining sample points in time could not be found. Accordingly, the length of the period of protection could not be determined with certainty.

### EXAMPLE 10

About 600 salmon (*Salmo salar*) weighing on average about 100 grams were divided into 4 groups, each of 150 fish. The groups 1-3 were injected with 3 different formulations comprising 25 mg of hexaflumuron per ml vaccine. All of the formulations were prepared on the basis of a vaccine against furunculosis, vibriosis, cold water vibriosis and winter ulcer, comprising inactivated bacteria of *Aeromonas salmonicida* subsp. *salmonicida, Vibrio anguillarum, Vibrio salmonicida* and *Vibrio viscosus.* Hexaflumuron was mixed/formulated into the vaccine in three different manners in order to test the effect of the formulation on the depot effect in the fish and on the stability characteristics of the vaccine emulsions. Group 4 was injected with a formulation that did not contain hexaflumuron and thus served as a negative control in respect of susceptibility for sea lice. The fish was injected while being kept in freshwater and transferred to sea water (mini-cages) 5 weeks after administration.

Following transfer to sea water, the fish were exposed to natural sea lice infestation. Every three weeks lice were counted on 10 fish of each group to assess if the number of lice between the various groups differed. The trial was terminated about 18 weeks after sea water transfer. The water temperature varied between 3 and 8°C throughout the trial. Figure 9 shows the average number of sea lice in each group throughout the trial. T1 and T2 are not shown in the figure as sea lice were not detected at these points in time. The figure shows that fish treated with hexaflumuron had significantly lower number of sea lice at 9 and 12 weeks after sea water transfer (T3 and T4). After termination of the sea lice recordings at 12 weeks after sea water transfer, the fish in all groups were deloused using the bath treatment composition ALPHA MAX (deltamethrin). Only the fish in the control group needed delousing, but as all groups were kept in the same cage, all groups were exposed to the bath treatments. The treatment was successful and at the counting at 15 weeks after delousing, sea lice were not found in any of the groups.

Based on the results shown in Figure 9 it can thus be concluded with certainty that in this trial, hexaflumuron protected the fish against recurrent infestation for at least 12 weeks after sea water transfer (18 weeks after injection). It is possible that the protection would be longer, but as the trial was terminated after 18 weeks without new sea lice infestation in the control group, this trial does not directly permit the conclusion that the protection can be extended beyond 12 weeks after sea water transfer of the fish.

## Claims

1. Hexaflumuron for controlling parasitic infestations in fish.

2. Use of hexaflumuron in the manufacture of a composition for controlling parasitic infestations in fish.

3. Use according to claim 2 wherein the composition is for oral administration of hexaflumuron.

4. Use according to claim 3 wherein the composition further comprises a component selected from the group consisting of components of fish feed, pre-manufactured fish feed and pellets.

5. Use according to any of claims 2-4 wherein the composition is suitable for administration to the fish by adding an effective amount hereof to the habitat of the fish.

6. Use according to claim 2 wherein the composition is an injectable composition.

7. Use of hexaflumuron in the manufacture of an injectable composition for simultaneous control of parasitic infestations and infectious diseases in fish, the composition further comprising an antigen conferring upon administration to the fish immunological protection against at least one viral or bacterial species.

8. Use according to any of claims 2-7 wherein the fish to which the injectable composition is administered is farmed fish.

9. Use according to claim 8 wherein the fish is selected from the group consisting of a salmonid species including Atlantic salmon (*Salmo salar*), rainbow trout (*Oncorhynchus mykiss*) and sea trout (*Salmo trutta*), and sea bass (*Dicentrarchus labrax).*

10. Use according to any of claims 2-9 wherein the injectable composition protects the fish against infestation with a parasitic crustacean species.

11. Use according to claim 10 wherein the crustacean species belongs to sea lice species including *Lepeophtheirus salmonis* and *Caligus elongatus.*

12. Use according to claim 10 wherein the crustacean species is an isopod species including *Anilocra physodes.*

13. Use according to any of claims 2-12 wherein the composition protects against parasitic infestation for a period of at least 12 weeks after administration of the composition.

14. A composition for the treatment and/or prophylaxis of parasitic infestations in fish, the composition comprising hexaflumuron.

15. A composition according to claim 14 for use as bath treatment composition.

16. A composition according to claim 14 which is a fish feed composition.

17. A composition according to claim 14 which is an injectable composition.

18. A composition according to claim 17 for simultaneous treatment and/or prophylaxis of parasitic infestations and infectious diseases in fish, said composition further comprising an immunologically active antigenic substance conferring upon administration to the fish protection against at least one viral or bacterial species.

19. A composition according to any of claims 14-18 wherein the fish to which the composition is administered is farmed fish.

20. A composition according to claim 19 wherein the fish is selected from the group consisting of a salmonid species including Atlantic salmon (*Salmo salar*), rainbow trout (*Oncorhynchus mykiss*) and sea trout (*Salmo trutta*) and sea bass (*Dicentrarchus labrax).*

21. A composition according to any of claims 14-20 which protects the fish against infestation with a parasitic crustacean species.

22. A composition according to claim 21 which protects the fish against infestation with a crustacean species belonging to sea lice species including *Lepeophtheirus salmonis* and *Caligus elongatus.*

23. A composition according to claim 21 which protects the fish against infestation with an isopod species including *Anilocra physodes.*

24. A composition according to any of claims 14-23 which protects the fish against parasitic infestation for a period of at least 12 weeks after administration of the composition.

25. A composition according any of claims 18-25 where the immunologically active antigenic substance is selected from the group consisting of an inactivated virus, an attenuated virus, a fragment of a virus, live bacterial cells, attenuated bacterial cells, non-viable bacterial cells and a bacterial cell component.

## Patentansprüche

1. Hexaflumuron zur Bekämpfung von parasitärem Befall bei Fischen.

2. Verwendung von Hexaflumuron in der Herstellung einer Zusammensetzung zur Bekämpfung von parasitärem Befall bei Fischen.

3. Verwendung nach Anspruch 2, wobei die Zusammensetzung für die orale Verabreichung von Hexaflumuron geeignet ist.

4. Verwendung nach Anspruch 3, wobei die Zusammensetzung weiter umfasst einen Bestandteil, ausgewählt aus der Gruppe, bestehend aus Bestandteilen von Fischfutter, vorgefertigtem Fischfutter und Pellets.

5. Verwendung nach einem der Ansprüche 2 bis 4, wobei die Zusammensetzung für die Verabreichung an Fischen durch Zugabe einer wirksamen Menge davon in den Lebensraum des Fisches geeignet ist.

6. Verwendung nach Anspruch 2, wobei die Zusammensetzung eine injizierbare Zusammensetzung ist.

7. Verwendung von Hexaflumuron in der Herstellung einer injizierbaren Zusammensetzung für das gleichzeitige Bekämpfen von parasitärem Befall und Infektionskrankheiten bei Fischen, wobei die Zusammensetzung weiter ein Antigen umfasst, das dem Fisch nach der Verabreichung immunologischen Schutz vor mindestens einer Virus- oder Bakterienspezies vermittelt.

8. Verwendung nach einem der Ansprüche 2 bis 7, wobei der Fisch, dem die injizierbare Zusammensetzung verabreicht wird, ein gezüchteter Fisch ist.

9. Verwendung nach Anspruch 8, wobei der Fisch ausgewählt ist aus der Gruppe, bestehend aus einer Spezies der Salmoniden einschließlich Atlantiklachs (*Salmo salar*), Regenbogenforelle (*Oncorhynchus mykiss*), und Meerforelle (*Salmo trutta*) und Wolfsbarsch (*Dicentrarchus labrax*).

10. Verwendung nach einem der Ansprüche 2 bis 9, wobei die injizierbare Zusammensetzung den Fisch vor Befall durch eine parasitäre Crustaceenspezies schützt.

11. Verwendung nach Anspruch 10, wobei die Crustaceenspezies zu einer Spezies der Meerläuse gehört, einschließlich *Lepeophtheirus salmonis* und *Caligus elongatus.*

12. Verwendung nach Anspruch 10, wobei die Crustaceenspezies einer Spezies der Isopoden angehört, einschließlich *Anilocra physodes.*

13. Verwendung nach einem der Ansprüche 2 bis 12, wobei die Zusammensetzung vor parasitärem Befall für einen Zeitraum von mindestens 12 Wochen nach Verabreichung der Zusammensetzung schützt.

14. Zusammensetzung zur Behandlung und/oder Prophylaxe von parasitärem Befall bei Fischen, wobei die Zusammensetzung Hexaflumuron umfasst.

15. Zusammensetzung nach Anspruch 14, zur Verwendung als Zusammensetzung für die Behandlung in einem Bad.

16. Zusammensetzung nach Anspruch 14, die eine Fischfutterzusammensetzung ist.

17. Zusammensetzung nach Anspruch 14, die eine injizierbare Zusammensetzung ist.

18. Zusammensetzung nach Anspruch 17 zur gleichzeitigen Behandlung und/oder Prophylaxe von parasitärem Befall und Infektionskrankheiten bei Fischen, wobei die Zusammensetzung weiter eine immunologisch wirksame, antigene Substanz umfasst, die dem Fisch nach Verabreichung Schutz vor mindestens einer Virus- oder Bakterienspezies vermittelt.

19. Zusammensetzung nach einem der Ansprüche 14 bis 18, wobei der Fisch, dem die Zusammensetzung verabreicht wird, ein gezüchteter Fisch ist.

20. Zusammensetzung nach Anspruch 19, wobei der Fisch ausgewählt ist aus der Gruppe, bestehend aus einer Spezies der Salmoniden einschließlich Atlantiklachs (*Salmo salar*), Regenbogenforelle (*Oncorhynchus mykiss*), und Meerforelle (*Salmo trutta*) und Wolfsbarsch (*Dicentrarchus labrax*).

21. Zusammensetzung nach einem der Ansprüche 14 bis 20, die den Fisch vor Befall durch eine parasitäre Crustaceenspezies schützt.

22. Zusammensetzung nach Anspruch 21, die den Fisch vor Befall durch eine Crustaceenspezies schützt, die einer Spezies der Meerläuse angehört, einschließlich *Lepeophtheirus salmonis* und *Caligus elongatus.*

23. Zusammensetzung nach Anspruch 21, die den Fisch vor Befall durch eine Isopodenspezies, einschließlich *Anilocra physodes,* schützt.

24. Zusammensetzung nach einem der Ansprüche 14 bis 23, die den Fisch vor parasitärem Befall für einen Zeitraum von mindestens 12 Wochen nach Verabreichung der Zusammensetzung schützt.

25. Zusammensetzung nach einem der Ansprüche 18 bis 25, worin die immunologisch wirksame, antigene Substanz ausgewählt ist aus der Gruppe, bestehend aus einem inaktivierten Virus, einem abgeschwächten Virus, einem Virus-Fragment, lebenden Bakterienzellen, abgeschwächten Bakterienzellen, nicht-lebensfähigen Bakterienzellen und einem Bestandteil einer Bakterienzelle.

## Revendications

1. Hexaflumuron pour contrôler les infestations parasitaires chez le poisson.

2. Utilisation d'hexaflumuron dans la fabrication d'une composition pour contrôler les infestations parasitaires chez le poisson.

3. Utilisation selon la revendication 2, dans laquelle la composition est destinée à une administration par voie orale d'hexaflumuron.

4. Utilisation selon la revendication 3, dans laquelle la composition comprend en outre un composé choisi dans le groupe constitué par les composés de nourriture pour poissons, les nourritures préfabriquées pour poissons et les granulés.

5. Utilisation selon l'une quelconques des revendications 2 à 4, dans laquelle la composition convient à une administration au poisson, en ajoutant une quantité efficace de celui-ci à l'habitat du poisson.

6. Utilisation selon la revendication 2, dans laquelle la composition est une composition injectable.

7. Utilisation d'hexaflumuron dans la fabrication d'une composition injectable pour le contrôle simultané des infestations parasitaires et des maladies infectieuses chez le poisson, la composition comprenant en outre un antigène conférant lors de l'administration au poisson, une protection immunologique contre au moins une espèce virale ou bactérienne.

8. Utilisation selon l'une quelconque des revendications 2 à 7, dans laquelle le poisson auquel la composition injectable est administrée, est un poisson d'élevage.

9. Utilisation selon la revendication 8, dans laquelle le poisson est choisi dans le groupe constitué par une espèce de salmonidé y compris le saumon de l'Atlantique (*Salmo salar*), la truite arc-en-ciel (*Oncorhynchus mykiss*) et la truite de mer (*Salmo trutta*) et le bar commun (*Dicentrarchus labrax*).

10. Utilisation selon l'une quelconques des revendications 2 à 9, dans laquelle la composition injectable protège le poisson contre une infestation avec une espèce de crustacée parasitaire.

11. Utilisation selon la revendication 10, dans laquelle l'espèce de crustacée appartient aux espèces de poux de mer incluant *Lepeophtheirus salmonis* et *Caligus elongatus.*

12. Utilisation selon la revendication 10, dans laquelle l'espèce de crustacée est une espèce d'isopode incluant *Anilocra physodes.*

13. Utilisation selon l'une quelconques des revendications 2 à 12, dans laquelle la composition protège contre l'infestation parasitaire pendant une période d'au moins 12 semaines après l'administration de la composition.

14. Composition pour le traitement et/ou la prophylaxie d'infestations parasitaires chez le poisson, la composition comprenant de l'hexaflumuron.

15. Composition selon la revendication 14, pour une utilisation en tant que composition de traitement en bain.

16. Composition selon la revendication 14, qui est une composition de nourriture de poisson.

17. Composition selon la revendication 14, qui est une composition injectable.

18. Composition selon la revendication 17 pour le traitement et/ou la prophylaxie simultanés d'infestations parasitaires et de maladies infectieuses chez le poisson, ladite composition comprenant en outre une substance antigénique immunologiquement active conférant lors de l'administration au poisson, une protection contre au moins une espèce virale ou bactérienne.

19. Composition selon l'une quelconques des revendications 14 à 18, dans laquelle le poisson auquel la composition est administrée est un poisson d'élevage.

20. Composition selon la revendication 19, dans laquelle le poisson est choisi dans le groupe constitué par une espèce de salmonidé y compris le saumon de l'Atlantique (*Salmo salar*), la truite arc-en-ciel (*Oncorhynchus mykiss*) et la truite de mer (*Salmo trutta*) et le bar commun (*Dicentrarchus labrax*).

21. Composition selon l'une quelconques des revendications 14 à 20, qui protège le poisson contre une infestation avec une espèce de crustacée parasitaire.

22. Composition selon la revendication 21, qui protège le poisson contre l'infestation avec une espèce de crustacée appartenant aux espèces de poux de mer incluant *Lepeophtheirus salmonis* et *Caligus elongatus.*

23. Composition selon la revendication 21, qui protège le poisson contre l'infestation avec une espèce d'isopode incluant *Anilocra physodes.*

24. Composition selon l'une quelconques des revendications 14 à 23, qui protège le poisson contre l'infestation parasitaire pendant une période d'au moins 12 semaines après l'administration de la composition.

25. Composition selon l'une quelconques des revendications 18 à 25, dans laquelle la substance antigénique immunologiquement active est choisie dans le groupe constitué d'un virus inactivé, un virus atténué, un fragment de virus, des cellules bactériennes vivantes, des cellules bactériennes atténuées, des cellules bactériennes non viables et un composé de cellule bactérienne.
